# EUROPÄISCHE PATENTANMELDUNG

(11) **EP 1 480 149 A2**
(43) Veröffentlichungstag der Anmeldung: **24.11.2004**
(21) Anmeldenummer: 04010483.8
(22) Anmeldetag: 03.05.2004
(51) Int. Cl.: G06F 19/00

(54) **Verfahren und eine Vorrichtung zur Verarbeitung und Ausgabe einer Versionsänderung eines medizinische Therapiehinweise umfassenden Datensatzes**

(30) Priorität: 20.05.2003 DE 10322684
(71) Anmelder: SIEMENS AKTIENGESELLSCHAFT, 80333 München (DE)
(72) Erfinder: Abraham-Fuchs, Klaus, 91058 Erlangen (DE); Rumpel, Eva, Dr., 91052 Erlangen (DE); Tiffe, Sven, 1020 Wien (AT)

(57) **Zusammenfassung**

Die Erfindung betrifft ein Verfahren zur Verarbeitung einer Versionsänderung eines medizinische Therapiehinweise (1) umfassenden Datensatzes(D), wobei der jeweilige einem Therapiehinweis (1) zugehörige Datensatz (D) eine Metadatenstruktur (M) mit einer Anzahl von Metadaten (MD) umfasst, welche auf ihren jeweiligen Aktualisierungsstatus geprüft werden, wobei anhand eines identifizierten geänderten Aktualisierungsstatus der betreffende Datensatz (D) aktiviert wird.

## Beschreibung

Die Erfindung betrifft ein Verfahren und eine Vorrichtung zur Verarbeitung und Ausgabe einer Versionsänderung eines medizinische Therapiehinweise umfassenden Datensatzes. Der Datensatz wird im Folgenden kurz selbst als Therapiehinweis bezeichnet. Der Datensatz kann sowohl Daten, also Therapiehinweise, als auch ausführbaren Code, also Softwarefragmente z. B. in Form von Expertenregeln, umfassen. Im allgemeinen sind in einer Datenbank mehrere Datensätze für verschiedene medizinische Therapiehinweise hinterlegt.

In den letzten Jahren gewinnen zunehmend so genannte medizinische Leitlinien (im englischen Sprachraum als Medical Practise Guidelines" oder "Clinical Practise Guidelines"), kurz Leitlinie oder Guideline genannt, an Bedeutung. Leitlinien sind diagnostische und/oder therapeutische Handlungs- und Entscheidungsempfehlungen (im Weiteren allgemein und zusammenfassend als Therapiehinweise bezeichnet) an den Arzt, die von übergeordneten und allgemein anerkannten Gremien der Ärzteschaft in einem breiten Konsens erarbeitet werden. Naturgemäß müssen solche Therapiehinweise ständig dem neuesten Stand des Wissens angepasst werden, und unterliegen somit einem ständigen, wenn auch meist lang- oder mittelfristigen Wandel. Typischerweise werden Therapiehinweise in Intervallen von wenigen Jahren neu überarbeitet und angepasst. Das heißt, ein Mediziner, also ein Arzt, Therapeut oder dergleichen, der Patienten bei der Diagnose- und Therapieentscheidung gemäß solcher etablierter Diagnose- bzw. Therapiehinweise behandelt, steht nun vor der Herausforderung, sich einen Überblick darüber zu verschaffen, welche Therapiehinweise gerade aktuell sind bzw. ob ein bereits zur Behandlung eingesetzter Therapiehinweis zwischenzeitlich geändert worden ist.

Hinsichtlich einer detaillierten Erläuterung von Therapiehinweisen sowie deren Abhängigkeiten und Wechselwirkungen wird auf die am gleichen Tage vom selben Anmelder eingereichte Patentanmeldung mit dem Titel "Verfahren zur Referenzierung von Therapiehinweise umfassenden Datensätzen" sowie "Verfahren zur Verknüpfung von medizinische Therapiehinweise umfassenden Datensätzen" verwiesen.

Zur Lösung dieser Probleme sind nach bestem Wissen der Anmelderin im Stand der Technik bisher keine automatisch ablauffähigen Verfahren bekannt geworden. Zudem ist der breite Einsatz von Therapiehinweisen, insbesondere in Form von Leitlinien, in der Medizin erst im Entstehen begriffen.

Die Erfindung bezieht sich auf eine Verbesserung der Einsatzmöglichkeiten von Therapiehinweise umfassende Datensätze, indem eine Möglichkeit aufgezeigt wird, die komplexen und umfassenden Datensätze vereinfacht und aktualisiert auszugeben und zu verteilen und damit eine auf den aktuellen Stand der Medizin basierende Behandlung von Patienten zu ermöglichen, insbesondere eine Vorrichtung zur automatischen Ausgabe von aktualisierten Datensätzen anzugeben.

Diese Aufgabe wird erfindungsgemäß mit den Merkmalen des Anspruchs 1 gelöst. Dazu ist bei einem Verfahren zur Verarbeitung und Ausgabe einer Versionsänderung eines medizinische Therapiehinweise umfassenden Datensatzes vorgesehen, dass dem jeweiligen einem Therapiehinweis zugehörigen Datensatz eine Metadatenstruktur mit einer Anzahl von Metadaten zugeordnet wird, welche auf ihren jeweiligen Aktualisierungsstatus geprüft werden, wobei anhand eines identifizierten geänderten Aktualisierungsstatus der betreffende Datensatz aktiviert wird, indem für den betreffenden Datensatz ein Warnhinweis erzeugt wird.

Ein Datensatz für einen Therapiehinweis umfasst in seiner allgemeinsten Form eine Sammlung von Daten - wie Eingangs- und/oder Ausgangsvariablen für Therapieinformationen und eine Anzahl von Expertenregeln - anhand derer auf der Basis von Therapieinformationen, z. B. anhand von Patientendaten und/oder anhand von Daten über verfügbare diagnostische und/oder therapeutische Mittel, mittels der Expertenregeln ein Therapiehinweis generiert wird. Die Therapieinformationen werden zur Generierung des Therapiehinweises in Form von Eingangs- oder Nutzdaten bereitgestellt.

Der Datensatz kann darüber hinaus mit einem Verweis oder einer Referenz versehen sein, anhand der beispielsweise auf einen nachfolgende Datensatz zur Generierung eines weiteren nachfolgenden Therapiehinweises oder anhand der auf nachfolgende Daten wie Therapieinformationen, z. B. auf weitere erforderliche Patientendaten und/oder erforderliche Expertenregeln, zur Ergänzung des betreffenden Therapiehinweises zugegriffen wird. Der Therapiehinweis wird anschließend anhand einer impliziten und/oder expliziten Metadatenstruktur mit einer Anzahl von Metadaten derart generiert und ausgegeben, dass die Metadaten in Form von grafischen und/oder alphanumerischen Informationen kurz und präzise ausgegeben werden. Dabei ist den jeweiligen Metadaten ein Aktualisierungsstatus zugeordnet, der bei der Ausgabe auf eine Änderung bzw. Aktualität geprüft wird, wobei ein identifizierter geänderter Aktualisierungsstatus zu einer Aktivierung eines entsprechenden Ausgangs des Datensatzes des Therapiehinweises in Form eines Warnhinweises führt.

Abhängig von dem jeweiligen Therapiehinweis, also dem jeweils aktivierten Ausgang, können sich unterschiedliche Möglichkeiten der Fortsetzung für den Therapiehinweis ergeben. Beispielsweise kann als Warnhinweis mittels des aktivierten Ausgangs ein Verteiler, z. B. ein elektronischer Postverteiler, zur Weiterleitung der Information über die Änderung des Therapiehinweises an weitere Benutzer generiert werden. Alternativ kann als Warnhinweis zumindest einem Ausgang des Datensatzes des betreffenden Therapiehinweises eine Referenz auf einen Datensatz eines im Behandlungsprozess nachfolgenden Therapiehinweis zugeordnet werden, so dass der nachfolgende Therapiehinweis über die Versionsänderung des vorangegangenen Therapiehinweises informiert wird.

Die Erfindung geht von der Erkenntnis aus, dass parallel und unabhängig von der Einführung von Leitlinien in der Medizin derzeit eine starke Ausweitung des Einsatzes moderner Informations- und Kommunikationstechnologie im Gesundheitswesen stattfindet. Der Einsatz elektronischer Datenverarbeitung im Krankenhaus (z. B. HIS = Hospital Information System, RIS = Radiology Information System, PACS = Picture Archive & Communication System, LIS = Laboratory Information System) und in der Arztpraxis (Praxisverwaltungssoftware, elektronische Patientenakte) wird mehr und mehr üblich. Von einem folgenden Entwicklungsschritt wird allgemein erwartet, dass eine Vernetzung dieser Software und Datenbanken über die Institutionen des Gesundheitswesens (Kliniken, Arztpraxen, Therapeutenpraxen etc.) hinweg stattfinden wird. Damit wird die Möglichkeit für ein "Vernetztes Gesundheitswesen", zuerst auf nationaler oder regionaler Ebene und später global geschaffen. Diese Entwicklung bietet die Basis zum Einsatz der beiden Aspekte der Erfindung.

Der Vorteil der Erfindung und ihrer Ausgestaltungen besteht insbesondere darin, dass komplette Behandlungsprozesse mit individuellen Behandlungsabfolgen auf ihre Gültigkeitsdauer und/oder ihre Aktualität prüfbar und überwachbar sind. Dies macht die Verwendung von Therapiehinweisen für Mediziner, also Ärzte, Therapeuten, etc., noch attraktiver. Insbesondere werden dabei Daten überwacht, welche üblicherweise in Art eines Impressums nur bei Aufruf angezeigt werden und somit beim täglichen Umgang mit dem Programm nicht angezeigt werden. Durch eine Überwachung von derartig "versteckten" Daten und/oder Informationen ist bei deren Änderung durch Aktivierung des Datensatzes eine einfache, sichere und schnelle Identifizierung von geänderten oder aktualisierten und/oder neu hinzugekommenen Daten oder Informationen ermöglicht.

Vorteilhafte Ausgestaltungen der Erfindung sind Gegenstand der Unteransprüche.

Unter Metadaten versteht man strukturierte Daten die die Eigenschaften von Datensätzen beschreiben und den inhaltlichen Kontext herstellen. Metadaten umfassen Informationen und Angaben, die es ermöglichen, einen Datensatz zu verstehen und austauschbar mit anderen Benutzern zu machen. Therapiehinweise beschreibende Metadaten können z. B. Titel, Autorenname, Veröffentlichungstag, Evidenzgrad sein. Metadaten in einem definierten maschinenlesbaren Format ermöglichen dabei eine automatische Identifizierung und Klassifizierung und gezielte Ausgabe einer Versionsänderung eines Therapiehinweise umfassenden Datensatzes. So weist z. B. die Änderung des Metadatums "Veröffentlichungstag" eindeutig auf eine Versionsänderung eines Therapiehinweises hin. Des Weiteren können dabei die Metadaten im vorgegebenen Format im Datensatz hinterlegt sein.

Bei einer weiteren möglichen Ausführungsform wird der Datensatz beim Einlesen von neuen Metadaten mit geänderten Aktualisierungsstatus in den Datensatz aktiviert. Beispielsweise wird der Datensatz oder ein den formell und/oder inhaltlich geänderten Therapiehinweis umfassendes Modul automatisch beim Einlesen des betreffenden geänderten Datensatzes bzw. Moduls in eine Datenbank aktiviert, wobei je nach Art und Aufbau der Datenbank der vorherige und nunmehr veraltete Datensatz überschrieben bzw. mit entsprechender Kennung archiviert wird. Anhand der Aktivierung des neuen geänderten Datensatzes mit dem aktuellen Therapiehinweis beispielsweise in Form eines gesetzten Ausgangs wird automatisch eine Benachrichtigung in Form eines elektronischen Nachrichten- und/oder Informationsverteilers, z. B. ein E-Mail-Verteiler, generiert und ausgegeben.

Alternativ oder zusätzlich kann beim Benutzen eines geänderten und nunmehr aktualisierten Datensatz anhand von Metadaten mit geänderten Aktualisierungsstatus der Datensatz aktiviert werden. Somit ist sichergestellt, dass beim Aufruf eines aktualisierten Therapiehinweises durch einen Benutzer, z. B. einem Arzt oder Therapeuten, anhand der Aktivierung des Datensatzes, z. B. in Form eines gesetzten Ausgangs, automatisch eine Anzeige oder Meldung mit einem entsprechenden Hinweis auf den geänderten Aktualisierungsstatus erfolgt. Ein derartig automatischer Änderungshinweis führt ohne zeitaufwendige Suche zu einem sicheren und stets aktuellen sowie genauen Umgang der Benutzer mit den momentan geltenden Therapiehinweisen.

In einer bevorzugten Ausführungsform werden die Metadaten mit geändertem Aktualisierungsstatus und/oder die durch diese Metadaten charakterisierten Teile des Datensatzes modifiziert ausgegeben. Zweckmäßigerweise werden die Metadaten mit geändertem Aktualisierungsstatus und/oder die durch diese Metadaten charakterisierten Teile des Datensatzes visuell modifiziert ausgegeben. Beispielsweise werden die in einem Fenster ausgegebenen Informationen zu den Metadaten grafisch z. B. durch eine entsprechende Farbe, einen Kontrast oder eine Umrahmung hervorgehoben. Dabei werden stets nur diejenigen Datenelemente der Metadaten grafisch hervorgehoben, welche sich seit der letzten Versionsänderung bzw. seit dem letzten Aktualisierungsstatus geändert haben. Alternativ oder zusätzlich können die Metadaten in einem separaten Feld ausgegeben werden. Bei einer weiteren bevorzugten Ausführungsform werden die Metadaten mit geändertem Aktualisierungsstatus und/oder die durch diese Metadaten charakterisierten Teile des Datensatzes audio-visuell modifiziert ausgegeben. Dabei wird zusätzlich zu der grafischen Animation der Metadaten bei deren Ausgabe auch ein Ton- und/oder Sprachsignal ausgegeben, durch welches der Benutzer des betreffenden Therapiehinweises auf eine Änderung in Form eines Meldesignals oder einer Sprachmeldung hingewiesen wird.

Je nach Umfang der Änderung des Therapiehinweises können die Metadaten mit geändertem Aktualisierungsstatus und/oder die durch diese Metadaten charakterisierten Teile des Datensatzes gruppiert oder separat ausgegeben werden. Alternativ oder zusätzlich, insbesondere zur Dokumentation der Kenntnisnahme der Änderungen durch einen Benutzer werden die Metadaten mit geändertem Aktualisierungsstatus vorteilhafterweise und/oder die durch diese Metadaten charakterisierten Teile des Datensatzes interaktiv modifiziert ausgegeben. Zur Kenntnisnahme des geänderten Aktualisierungsstatus wird zweckmäßigerweise eine Bestätigungsanforderung interaktiv ausgegeben. Dabei wird bei Kenntnisnahme des geänderten Therapiehinweises durch einen Nutzer anhand der ausgegebenen Bestätigungsanforderung eine Bestätigung eingegeben. Vorzugsweise wird zur Dokumentation dieser Bestätigung für die Kenntnisnahme die Bestätigung der Kenntnisnahme im betreffenden Datensatz hinterlegt. Alternativ oder zusätzlich kann die Bestätigung auch zentral in einer zentralen Datei hinterlegt werden.

Bedingt durch sich häufig ändernde Metadaten und eine daraus resultierende modifizierte Ausgabe der betreffenden Metadaten kann es bei der Ausgabe eines mehrfach geänderten Therapiehinweises für einen Nutzer unübersichtlich werden. Zur Vermeidung dessen wird vorzugsweise anhand der durch den Nutzer eingegebenen Bestätigung die modifizierte Ausgabe der betreffenden Metadaten und/oder die modifizierte Ausgabe von durch diese Metadaten charakterisierten Teilen rückgesetzt. Je nach Art und Aufbau der Datenbank bzw. des jeweiligen Datensatzes kann das Rücksetzen der modifizierten Ausgabe von betreffenden Metadaten und/oder von durch diese Metadaten charakterisierten Teilen benutzerbezogen und/oder zeitbezogen und/oder ereignisbezogen ausgeführt werden. Das heißt, das Rücksetzen der modifizierten Metadaten erfolgt für jeden einzelnen Benutzer separat. Alternativ oder zusätzlich kann die Rücksetzung nach Ablauf einer Zeit, z. B. nach Ablauf von einem Monat nach Einlesen des geänderten Datensatzes ausgeführt werden. Auch kann das Rücksetzen anhand eines Maximalwertes für die Anzahl von geänderten und modifiziert ausgegebenen Metadaten ausgeführt werden. Beispielsweise dürfen maximal fünf verschiedene Änderungen gleichzeitig modifiziert ausgegeben werden. Mit anderen Worten: Die modifizierte Ausgabe der betreffenden Metadaten wird vorteilhafterweise auch nach Ablauf einer vorgegebenen Zeit und/oder ereignisgesteuert rückgesetzt.

Um insbesondere eine schnelle, die Änderung berücksichtigende Benutzung von aktualisierten Therapiehinweisen, insbesondere in großen Institutionen wie beispielsweise in Krankenhäusern zu ermöglichen, wird zweckmäßigerweise bei einem identifizierten geänderten Aktualisierungsstatus automatisch oder ereignisgesteuert an aktuelle und/oder hinterlegte Nutzer des betreffenden Therapiehinweises eine Informationsmeldung ausgegeben. Eine Anwendung des oben beschriebenen Verfahrens sowohl zur Identifizierung als auch zur Ausgabe von aktualisierten Therapiehinweisen erfolgt vorteilhaft auch zur Schulung und Weiterbildung von Nutzern, wie Therapeuten oder Ärzten.

Nachfolgend wird ein Ausführungsbeispiel der Erfindung anhand der Zeichnung näher erläutert. Einander entsprechende Gegenstände oder Elemente sind in allen Figuren mit den gleichen Bezugszeichen versehen.

Darin zeigen:
- FIG 1: schematisch einen Aufbau eines mindestens einen Therapiehinweis umfassenden Datensatzes, und
- FIG 2 bis 3: eine schematische Darstellung verschiedener alternativer Ausführungsformen für eine Ausgabe von mindestens einen Therapiehinweis umfassenden Datensatz.

Einander entsprechende Teile sind in allen Figuren mit den gleichen Bezugszeichen versehen.

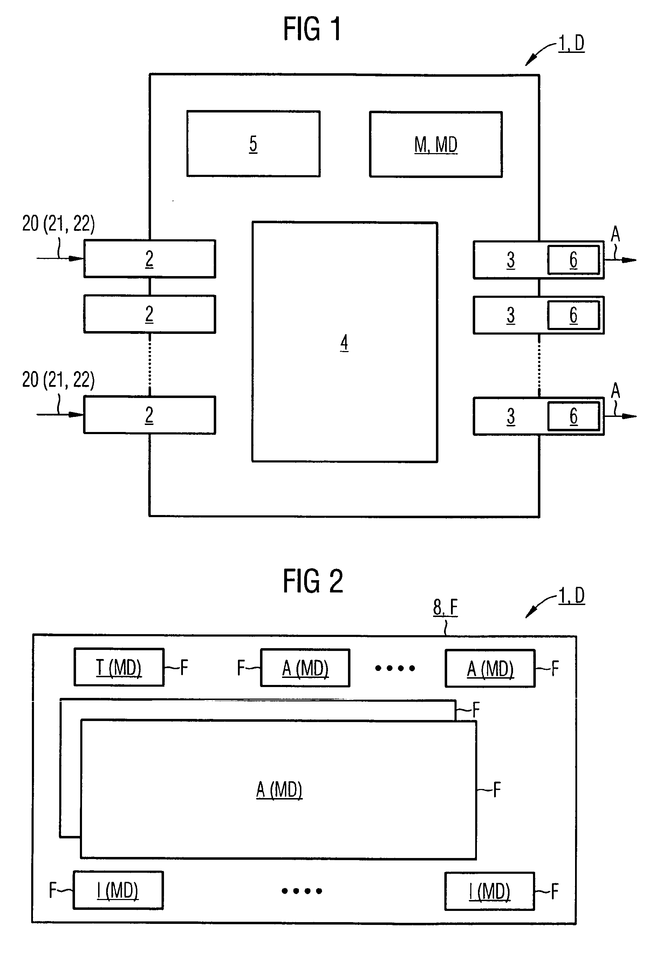

FIG 1 zeigt eine schematische Darstellung der softwaremäßigen Realisierung der Grundstruktur einer medizinischen Leitlinie, anhand eines Datensatzes D für einen Therapiehinweis 1. Der Datensatz D (im Weiteren nur kurz Therapiehinweis 1 genannt) umfasst zumindest einen Eingang 2 (auch Eingangsvariable genannt) und zumindest einen Ausgang 3. Über den oder jeden Eingang 2 werden dem Therapiehinweis 1 Therapieinformationen 20, wie z. B. Patientendaten 21 oder Informationen 22, über diagnostische und/oder therapeutische Mittel, direkt oder indirekt zugeführt. Den Kern des Therapiehinweises 1 bildet eine Expertenregel 4. Anhand der Expertenregel 4, welche wiederum anhand einer Metadatenstruktur M mit einer Mehrzahl von Metadaten MD charakterisiert ist, werden aufgrund der Therapieinformationen 20 an dem oder jedem Eingang 2 einzelne oder mehrere Ausgänge 3 aktiviert und damit individuelle, d. h. patienten- bzw. mittelbezogene Handlungs- und/oder Entscheidungsempfehlungen A in Form von Vorgehensweisen oder Handlungsanweisungen ausgegeben.

Die Therapieinformationen 20 an einem Eingang 2, also die Patientendaten 21 oder Informationen 22, können von verschiedenen Kategorien sein, z. B. Messdaten wie Blutdruck, Elektrokardiogramm, verfügbare technische Mittel, wie Röntgengerät, etc., Erkenntnisse wie eine vorausgehende Diagnose oder Handlungen wie eine durchgeführte Therapieoption, etc. Ebenso können die an den Ausgängen 3 ausgegebenen individuellen Handlungs- und/oder Entscheidungsempfehlungen A von verschiedenen Kategorien sein, z. B. eine Erkenntnis wie eine aus den Eingangswerten der Therapieinformation 20 abgeleitete Diagnose, eine Handlungsempfehlung oder aus Eingangsdaten der Therapieinformation 20 abgeleitete Untersuchungsergebnisse.

Die Expertenregel 4 ist im Allgemeinen eine so genannte Inferenz-Regel, mit der aus den Therapieinformationen 20 (auch Eingangsdaten genannt) einzelne oder mehrere individuelle - patienten- und/oder mittelbezogene - Handlungs- und/oder Entscheidungsempfehlungen A abgeleitet werden, die zur Aktivierung des jeweiligen Ausgangs 3 oder der jeweiligen Ausgänge 3 führen und an diesem ggf. ausgegeben werden.

Im Ausführungsbeispiel kann darüber hinaus jeder Therapiehinweis 1 ein den Therapiehinweis 1 eindeutig kennzeichnendes Ordnungsmerkmal 5 umfassen. Das Ordnungsmerkmal 5 ist geeignet und vorgesehen, einen Therapiehinweis 1, der z. B. in einer zentralen Datenbank (nicht dargestellt) gespeichert ist und in einer medizinischen Institution verwendet wird, zu referenzieren.

Zur Identifizierung einer Änderung, z. B. einer formellen und/oder inhaltlichen Änderung des Therapiehinweises 1, wird beim Einlesen oder Generieren des Therapiehinweises 1 dieser anhand der Metadatenstruktur M mit Metadaten MD auf deren Aktualisierungsstatus hin analysiert. Dabei wird anhand eines identifizierten geänderten Aktualisierungsstatus eines der Metadaten MD der Datensatz D und somit der Therapiehinweis 1 aktiviert, indem als Warnhinweis beispielsweise ein Ausgang A gesetzt wird. Anhand des gesetzten Ausgangs A kann beispielsweise automatisch eine Benachrichtigung über den geänderten und aktuellen Therapiehinweis 1 in Form eines elektronischen Nachrichten- und/oder Informationsverteilers, z. B. eines E-Mail-Verteilers, generiert und an weitere Nutzer ausgegeben werden. Somit kann die Identifizierung und Ausgabe der Änderung eines Therapiehinweises 1 unmittelbar in Arbeitsabläufe von größeren Institution mit miteinander verbundenen Ärzten und/oder Therapeuten eingebunden werden, so dass innerhalb eines derartigen Verbunds oder Netzes automatisch eine Benachrichtigung der diesen geänderten Therapiehinweis 1 anwendenden Nutzern erfolgt bzw. bei Aufruf und somit Benutzen eines geänderten Therapiehinweises 1 durch einen der Nutzer in diesem Verbund oder in diesem Netz automatisch anhand der Aktivierung des Datensatzes D mittels des Ausgangs A eine Meldung ausgegeben wird.

Unter Metadaten MD werden in einer Metadatenstruktur M strukturierte Daten verstanden, die die Eigenschaften des jeweiligen Datensatzes D des betreffenden Therapiehinweises 1 näher beschreiben und den inhaltlichen Kontext herstellen. Metadaten MD umfassen beispielsweise Informationen und Angaben, die es ermöglichen, einen Datensatz D zu verstehen und austauschbar mit anderen Benutzern zu machen. Therapiehinweise 1 beschreibende Metadaten MD können z. B. Titel, Autorenname, Veröffentlichungstag, Evidenzgrad sein. Die Metadaten MD werden dazu in einem definierten maschinenlesbaren Format im Datensatz D hinterlegt und ermöglichen dabei eine automatische Identifizierung und Klassifizierung und gezielte Ausgabe einer Versionsänderung des Datensatzes D des betreffenden Therapiehinweises 1. So weist z. B. die Änderung des Metadatums MD "Veröffentlichungstag" eindeutig auf eine Versionsänderung eines Therapiehinweises 1 hin.

Üblicherweise handelt es sich bei den Metadaten MD um so genannte "versteckte" Informationen oder Daten, die anhand der den Metadaten MD zugehörigen Aktualisierungsstatus automatisch auf eine Änderung überprüft werden, so dass bei einer identifizierten Änderung der Therapiehinweis 1 automatisch aktiviert wird. Unter versteckten Daten oder Informationen werden beispielsweise Impressumsinformationen, wie Änderung der Auflage oder Version oder der Name des Autors des Therapiehinweises 1 verstanden. Diese Informationen werden üblicherweise nicht unmittelbar auf einem Bildschirm ausgegeben. Vielmehr sind diese zumeist in einem aufrufbaren Feld oder in einer aufrufbaren Anzeige hinterlegt, so dass im alltäglichen Umgang diese Informationen oder Daten nicht auf Aktualität überprüft werden. Hier setzt die automatische Überprüfung der im Datensatz D enthaltenen und den Therapiehinweis 1 näher beschreibenden, aber nicht ausgegebenen Daten mittels der zugehörigen Metadaten MD an, so dass bei Änderung solch "versteckter" Daten automatisch eine Aktivierung des betreffenden Therapiehinweises 1 ausgeführt wird und zum Beispiel automatisch eine Meldung oder Benachrichtigung von betreffenden Nutzern erfolgt.

D.h. je nach Funktion und Aufbau des jeweiligen Ausgangs 3 kann dieser bei Aktivierung anhand der geänderten Metadaten MD zum einen eine modifizierte Ausgabe von Handlungs- und/oder Entscheidungsempfehlungen A in Form der Ausgabe von modifizierten Metadaten MD und zum anderen eine Referenz 6 zur Referenzierung eines weiteren Therapiehinweises 1 bewirken. Wenn mit der Aktivierung eines Ausgangs 3 keine Ausgabe bzw. kein weiterer Therapiehinweis 1 verknüpft werden soll, wird der Wert der Referenz 6 auf einen vorgegebenen Wert, z. B. Null, gesetzt. Wenn mit der Aktivierung eines Ausgangs 3 eine Ausgabe bzw. ein nachfolgender Therapiehinweis 1 verknüpft werden soll, wird ein entsprechendes Ausgabemittel 8, wie in FIG 2 gezeigt, angesteuert bzw. als Referenz 6 dessen Ordnungsmerkmal 5 eingetragen.

FIG 2 zeigt ein Beispiel für eine Ausgabe von Metadaten MD eines zugehörigen Therapiehinweises 1 auf einem Ausgabemittel 8. Als Ausgabemittel 8 dient beispielsweise ein Bildschirm oder ein Drucker. Für eine audio-visuelle Ausgabe von geänderten Metadaten MD, insbesondere von diesen zugrunde liegenden Informationen oder Daten ist beispielsweise ein nicht näher dargestellter Lautsprecher vorgesehen, über welchen ein Audiosignal in Form einer Sprachmeldung oder eines Tons ausgegeben wird. Alternativ kann auch eine Meldung in Form einer elektronischen Nachricht, z.B. als Email ausgegeben werden.

Dabei werden auf dem Ausgabemittel 8 die verschiedenen den jeweiligen Metadaten MD zugrunde liegenden Informationen oder Daten, z. B. der Titel T des Therapiehinweises 1, der/die Autor/en N, oder weitere Informationen I, wie Ausgabedatum des Therapiehinweises 1, Evidenzgrad der Handlungs- und/oder Entscheidungsempfehlung A, auf einem Bildschirm oder Drucker in entsprechenden Feldern oder Anzeigefenstern F einer Anzeige ausgegeben. Dabei stellt die Anzeige gemäß FIG 2 nur eine mögliche Anordnung der Anzeigefenster F dar. Je nach Art, Inhalt, Umfang und Anzahl der anzuzeigenden Therapiehinweise 1 kann eine beliebig andere Anordnung sowie Größe der Anzeigefenster F vorgesehen sein.

Um einem Nutzer die auf dem Ausgabemittel 8 ausgegebenen Anzeigefenster F und deren zugehörigen Aktualisierungsstatus leicht und sicher identifizieren zu können, d. h. um einem Nutzer eine mögliche Versionsänderung - sowohl formell als auch inhaltlich - wie beispielsweise Änderung der Gültigkeitsdauer oder eines Verfallsdatums, Änderung der Namen des/der Autors/en, Änderung der Priorisierung von Handlungs- und Entscheidungsempfehlung A, Evidenzgrad dieser Handlungs- und Entscheidungsempfehlung A, des zugrunde liegenden Therapiehinweises 1 - anzuzeigen, werden bei der Generierung und der anschließenden Ausgabe der den Metadaten MD zugrunde liegenden und den Therapiehinweis 1 repräsentierenden Informationen oder Daten die Metadaten MD dahingehend analysiert und entsprechend aktiviert, dass bei einen geänderten Aktualisierungsstatus aufweisenden Metadaten MD diese entsprechend modifiziert anhand der zugehörigen Informationen bzw. Daten ausgegeben werden.

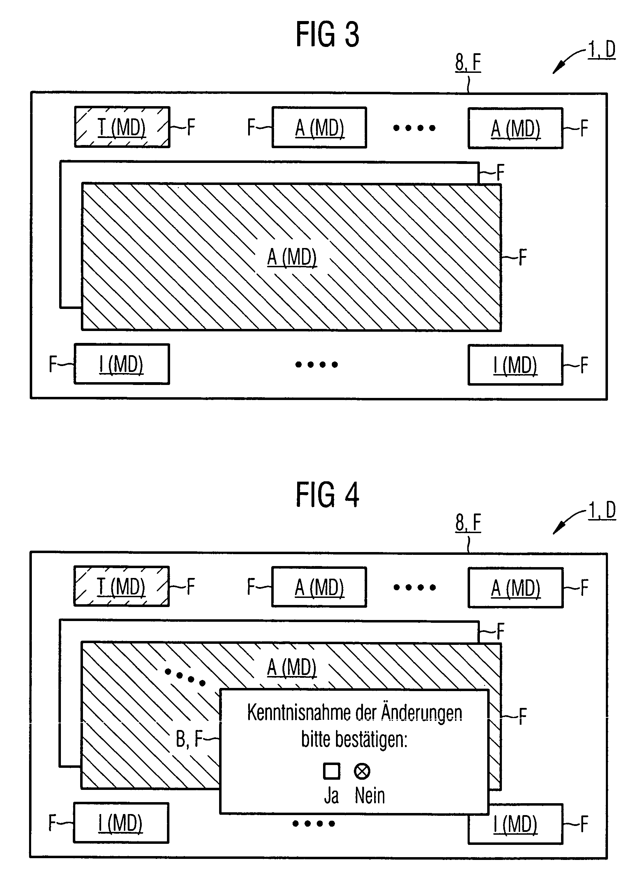

In FIG 3 ist beispielhaft eine modifizierte Ausgabe von Metadaten MD, und daneben eine modifizierte Ausgabe einer einen neuen Aktualisierungsstatus aufweisenden Handlungs- und Entscheidungsempfehlung A in Form eines visuell, insbesondere grafisch beispielsweise durch ein Linienmuster hervorgehobenen Anzeigefensters F dargestellt. Beispielsweise können die geänderten Metadaten MD, insbesondere die diesen zugrunde liegenden Informationen oder Daten auch durch eine entsprechende Farbe, durch einen Kontrast und/oder eine Umrahmung bei deren Ausgabe visuell hervorgehoben werden.

Je nach Anzahl und Art der einen geänderten Aktualisierungsstatus aufweisenden Metadaten MD können auch mehrere Anzeigefenster F modifiziert ausgegeben. Beispielsweise ist in der FIG 3 auch das Anzeigefenster F für den Titel T, welcher sich mit der neuen Version geändert hat, entsprechend grafisch hervorgehoben. Zusätzlich oder alternativ kann auch bei Generierung des Therapiehinweises 1 und anschließender Ausgabe auf dem Ausgabemittel 8 ein Signal, z. B. ein Tonsignal oder eine Sprachmeldung ausgegeben werden, um audio-visuell eine Änderung des Aktualisierungsstatus auszugeben. Hierzu wird mittels eines Ausgangs A des Therapiehinweises 1 ein entsprechendes Steuersignal einem Lautsprecher oder einer anderen Audioeinheit zugeführt.

Je nach Art und Vorgabe können bei der modifizierten Ausgabe der den Metadaten MD zugrunde liegenden Informationen oder Daten zur besseren Übersichtlichkeit der Anzeige lediglich jene Metadaten MD anhand der zugehörigen Informationen oder Daten visuell und/oder audio-visuell modifiziert ausgegeben werden, welche sich seit dem letzten, d. h. vorangegangenen Aktualisierungsstatus geändert haben. Auch können geänderte Metadaten MD, insbesondere deren Informationen oder Daten gruppiert in einem gemeinsamen Anzeigefenster F oder separat ausgegeben werden.

FIG 4 zeigt eine weitere modifizierte Ausgabe von Metadaten MD zur Identifizierung und Anzeige der Änderung des zugrunde liegenden Aktualisierungsstatus. Hierbei wird zur Ausgabe der Änderung des Aktualisierungsstatus der Metadaten MD vorzugsweise ein Anzeigefenster F interaktiv modifiziert ausgegeben. Das heißt, zusätzlich zu der eigentlichen Ausgabe der den Metadaten MD zugrunde liegenden Informationen oder Daten im zugehörigen Anzeigefenster F kann eine Bestätigungsanforderung B durch Öffnen eines zugehörigen Anzeigefensters F ausgegeben werden, in welchem der Nutzer des geänderten Therapiehinweises 1 die Kenntnisnahme des geänderten Therapiehinweises 1 bestätigen muss, indem er eine entsprechende Eingabe im zugehörigen Anzeigefenster F ausführt. Vorteilhafterweise wird zur Dokumentation der Eingabe der Bestätigung für die Kenntnisnahme des geänderten Therapiehinweises 1 durch den Nutzer die Bestätigung im zugehörigen Datensatz D und/oder in einem zentralen nicht näher dargestellten Datenspeicher hinterlegt.

Um eine durch eine zu hohe Anzahl von modifizierten Metadaten MD unübersichtliche Anzeige zu vermeiden, ist vorgesehen, dass beispielsweise mit der Bestätigungseingabe für die Kenntnisnahme durch den Nutzer die visuell und/oder audio-visuell hervorgehobene Ausgabe der betreffenden Informationen oder Daten der Metadaten MD zurückgesetzt wird. D.h. lediglich die modifizierte Ausgabe, insbesondere Darstellung wird auf eine Normalausgabe zurückgesetzt. Der geänderte und/oder aktualisierte Inhalt der betreffenden Informationen oder Daten bleibt erhalten.

Alternativ oder zusätzlich kann die hervorgehobene Ausgabe der Metadaten MD nach Ablauf einer vorgegebenen Zeit, z. B. nach Ablauf von einem Monat nach Änderung des Therapiehinweises 1 und/oder ereignisgesteuert, z. B. bei Überschreiten einer maximalen Anzahl von geänderten Metadaten MD für einen oder mehrere Therapiehinweise 1 rückgesetzt werden. Auch hierbei wird lediglich die formelle, d.h. die modifizierte Ausgabe rückgesetzt auf eine normale und übersichtliche Ausgabe, die inhaltliche Änderungen der betreffenden Informationen und Daten sowie der zugehörigen Metadaten MD bleibt erhalten. Unter Rücksetzung kann dabei auch verstanden werden, dass die üblicherweise versteckten Daten und Informationen nur für eine gewisse Zeit oder während einer Bestätigungsabfrage zu ihrer Kenntnisnahme ausgegeben und nach Ablauf der Zeit bzw. nach deren Bestätigung wieder versteckt im Datensatz D hinterlegt werden.

Bei einem bevorzugten Anwendungsfall des erfindungsgemäßen Verfahrens wird dieses zur Schulung und Weiterbildung beispielsweise als Lernsoftware eingesetzt. Dabei können zusätzliche Anzeigefenster F in Form von interaktiven Feldern generiert und ausgegeben werden, anhand derer Fragen und Antworten zum Verständnis von Änderungen des Therapiehinweises 1 interaktiv vom Nutzer aufgerufen und beantwortet werden können. Auch können Anzeigefenster F mit Gegenüberstellungen von alten und neuen Metadaten MD des Therapiehinweises 1 generiert und ausgegeben werden. Alternativ oder zusätzlich können durch einen implementierten Suchalgorithmus anhand des jeweiligen Aktualisierungsstatus diejenigen Metadaten MD identifiziert und gemeinsam, gruppiert und/oder seriell ausgegeben werden, welche sich seit dem letzten oder zu einem vorgegebenen, vorangegangenen Aktualisierungsstatus geändert haben.

## Patentansprüche

1. Verfahren zur Verarbeitung und Ausgabe einer Versionsänderung eines medizinische Therapiehinweise (1) umfassenden Datensatzes (D), wobei dem jeweiligen einem Therapiehinweis (1) zugehörige Datensatz (D) eine Metadatenstruktur (M) mit einer Anzahl von Metadaten (MD) zugeordnet wird, welche auf ihren jeweiligen Aktualisierungsstatus geprüft werden, wobei anhand eines identifizierten geänderten Aktualisierungsstatus für den betreffenden Datensatz (D) ein Warnhinweis erzeugt wird.

2. Verfahren nach Anspruch 1, wobei als Warnhinweis auf einem Ausgabemittel (8) ein Anzeigefenster (F) generiert, aktiviert und ausgegeben wird.

3. Verfahren nach Anspruch 1 oder 2, wobei als Warnhinweis eine elektronische Nachricht erzeugt und übertragen wird.

4. Verfahren nach einem der Ansprüche 1 bis 3, wobei als Warnhinweis ein akustisches und/oder optisches Signal erzeugt und ausgegeben wird.

5. Verfahren nach einem der Ansprüche 2 bis 4, wobei der Datensatz (D) beim Einlesen von neuen Metadaten (MD) mit geändertem Aktualisierungsstatus in den Datensatz (D) auf dem Ausgabemittel (8) aktiviert wird.

6. Verfahren nach einem der Ansprüche 2 bis 5, wobei beim Benutzen des Datensatzes (D) anhand von Metadaten (MD) mit geändertem Aktualisierungsstatus der Datensatz (D) auf dem Ausgabemittel (8) aktiviert wird.

7. Verfahren nach einem der vorangegangenen Ansprüche, wobei Metadaten (MD) mit geändertem Aktualisierungsstatus und/oder die durch diese Metadaten (MD) charakterisierten Teile des Datensatzes (D) modifiziert auf dem Ausgabemittel (8) ausgegeben werden.

8. Verfahren nach einem der vorangegangenen Ansprüche, wobei Metadaten (MD) mit geändertem Aktualisierungsstatus und/oder die durch diese Metadaten (MD) charakterisierten Teile des Datensatzes (D) visuell modifiziert auf dem Ausgabemittel (8) ausgegeben werden.

9. Verfahren nach einem der vorangegangenen Ansprüche, wobei Metadaten (MD) mit geändertem Aktualisierungsstatus und/oder die durch diese Metadaten (MD) charakterisierten Teile des Datensatzes (D) audio-visuell modifiziert auf dem Ausgabemittel (8) ausgegeben werden.

10. Verfahren nach einem der vorangegangenen Ansprüche, wobei Metadaten (MD) mit geändertem Aktualisierungsstatus und/oder die durch diese Metadaten (MD) charakterisierten Teile des Datensatzes (D) gruppiert oder separat auf dem Ausgabemittel (8) ausgegeben werden.

11. Verfahren nach einem der vorangegangenen Ansprüche, wobei Metadaten (MD) mit geändertem Aktualisierungsstatus und/oder die durch diese Metadaten (MD) charakterisierten Teile des Datensatzes (D) interaktiv modifiziert auf dem Ausgabemittel (8) ausgegeben werden.

12. Verfahren nach Anspruch 11, wobei zur Kenntnisnahme des geänderten Aktualisierungsstatus eine Bestätigungsanforderung (B) interaktiv auf dem Ausgabemittel (8) ausgegeben wird.

13. Verfahren nach Anspruch 11 oder 12, wobei anhand der ausgegebenen Bestätigungsanforderung (B) bei Kenntnisnahme eine Bestätigung durch einen Nutzer auf dem Ausgabemittel (8) eingegeben wird.

14. Verfahren nach Anspruch 13, wobei die Bestätigung der Kenntnisnahme im betreffenden Datensatz (D) hinterlegt wird.

15. Verfahren nach Anspruch 13 oder 14, wobei anhand der durch den Nutzer eingegebenen Bestätigung die modifizierte Ausgabe der betreffenden Metadaten (MD) und/oder von durch diese Metadaten (MD) charakterisierten Teile auf dem Ausgabemittel (8) rückgesetzt wird.

16. Verfahren nach einem der vorangegangenen Ansprüche, wobei nach Ablauf einer vorgegebenen Zeit und/oder ereignisgesteuert die modifizierte Ausgabe der betreffenden Metadaten (MD) und/oder von durch diese Metadaten (MD) charakterisierten Teile auf dem Ausgabemittel (8) rückgesetzt wird.

17. Verfahren nach einem der vorangegangenen Ansprüche, wobei bei einem identifizierten geänderten Aktualisierungsstatus automatisch oder ereignisgesteuert an aktuelle und/oder hinterlegte Nutzer des betreffenden Therapiehinweises (1) eine Informationsmeldung auf dem Ausgabemittel (8) ausgegeben wird.

18. Verfahren nach einem der vorangegangenen Ansprüche, wobei die Metadaten (MD) in einem vorgegebenen Format als den betreffenden Therapiehinweis (1) repräsentierende Informationen (I), insbesondere Titel (T), Autorname (N), Veröffentlichungstag, priorisierte Therapieempfehlung, im Datensatz (D) hinterlegt werden.

19. Verwendung des Verfahrens nach einem der Ansprüche 1 bis 18 zur Schulung und Weiterbildung von Nutzern von Therapiehinweise (1) umfassenden Datensätzen (D).

20. Vorrichtung zur Durchführung des Verfahrens zur Verarbeitung und Ausgabe einer Versionsänderung eines medizinische Therapiehinweise (1) umfassenden Datensatzes (D) nach einem der Ansprüche 1 bis 18, umfassend ein Ausgabemittel (8) zur Ausgabe eines Warnhinweises bei einer Versionsänderung des Datensatzes (D) anhand eines identifizierten geänderten Aktualisierungsstatus.

21. Vorrichtung nach Anspruch 20, bei der das Ausgabemittel (8) als ein Drucker, ein Bildschirm, ein Lautsprecher und/oder ein Modem ausgebildet ist.
